# EUROPÄISCHE PATENTSCHRIFT

(11) **EP 1 309 574 B1**
(45) Veröffentlichungstag und Bekanntmachung des Hinweises auf die Patenterteilung: **08.06.2005**
(21) Anmeldenummer: 01969547.7
(22) Anmeldetag: 30.07.2001
(51) Int. Cl.: C07D 277/32

(54) **VERFAHREN ZUR REINIGUNG VON 2-CHLOR-5-CHLORMETHYLTHIAZOL**
METHOD FOR PURIFYING 2-CHLORO-5-CHLOROMETHYL THIAZOLE
PROCEDE DE PURIFICATION DE 2-CHLORO-5-CHLOROMETHYLTHIAZOLE

(30) Priorität: 10.08.2000 DE 10038977
(43) Veröffentlichungstag der Anmeldung: 14.05.2003
(73) Patentinhaber: Bayer CropScience AG, 40789 Monheim (DE)
(72) Erfinder: DECKER, Matthias, 50674 Köln (DE); STEINBACH, Dimitry, 50859 Köln (DE); TASCHNER, Torsten, 51375 Leverkusen (DE)
(86) Internationale Anmeldenummer: PCT/EP2001/008789
(87) Internationale Veröffentlichungsnummer: WO 2002/012209

(56) Entgegenhaltungen:
- EP-A- 0 763 531
- EP-A- 0 794 180
- GB-A- 1 083 910

## Beschreibung

Die vorliegende Erfindung betrifft ein Verfahren zur Reindarstellung von 2-Chlor-5-chlormethylthiazol (CCMT) durch Destillation unter Zusatz von oligomeren Polyethern.

CCMT wird beispielsweise als Zwischenprodukt für die Herstellung von Insektiziden verwendet. Es besteht daher ein Bedarf, sehr reines CCMT mit hohen Ausbeuten herzustellen.

Aus EP-B 0 446 913 und EP-B 0 763 531 ist die Herstellung von CCMT mit anschließender Reinigung durch fraktionierende Destillation bzw. Reinigung durch einfache Destillation mit anschließender Kristallisation bekannt.

Nachteilig ist bei diesen Verfahren, dass bei der Destillation von CCMT wegen des hohen Siedepunkts bereits Zersetzung stattfindet, die zu einem nicht mehr handhabbaren festen Sumpf führt. Dies lässt sich nur vermeiden, indem man genügend CCMT im Sumpf belässt, um ihn flüssig zu halten, was zu Minderausbeuten führt.

Darüber hinaus ist bei fraktionierender Destillation die thermische Belastung höher als bei der einfachen Destillation, was zu größerem Produktverlust durch Zersetzung führt. Bei der einfachen Destillation ist eine anschließende Umkristallisation erforderlich, um hohe Produktreinheit zu erreichen. Dieser weitere Verfahrensschritt fährt zu zusätzlichem Ausbeuteverlust über die Mutterlauge.

Aufgabe der vorliegenden Erfindung war es, ein verbessertes destillatives Reinigungsverfahren für CCMT zu finden.

Überraschenderweise wurde jetzt gefunden, dass man CCMT in sehr guter Ausbeute und in sehr reiner Form erhält, wenn man rohes CCMT in Gegenwart eines oligomeren Polyethers destilliert.

Die vorliegende Erfindung betrifft demnach ein Verfahren zur Reinigung von CCMT, das dadurch gekennzeichnet ist, dass man CCMT in Gegenwart eines oligomeren Polyethers destilliert.

Überraschenderweise führt die einfache Destillation von CCMT in Anwesenheit eines oligomeren Polyethers zu höheren Ausbeuten und zu einem Destillat höherer Reinheit als ohne diesen Zusatz. Außerdem bleibt der Sumpf durch den Polyether-Zusatz flüssig und kann nach der Destillation abgelassen werden.

Die einfache Destillation kann diskontinuierlich oder kontinuierlich erfolgen.

Als Polyether kommen insbesondere oligomere aliphatische Polyether mit ein oder zwei endständigen Hydroxygruppen in Frage, bevorzugt können Polyethylenglykol oder Polypropylenglykol, besonders bevorzugt Polyethylenglykol jeweils mit einer mittleren Molmasse im Bereich von 200 - 3000 Dalton, bevorzugt im Bereich von 300 - 600 Dalton, besonders bevorzugt mit einer mittleren Molmasse von 400 Dalton, verwendet werden.

Die zugesetzte Polyethermenge kann beim erfindungsgemäßen Verfahren innerhalb eines größeren Bereichs variiert werden. Im allgemeinen liegt sie zwischen der 0.01 fachen und 10fachen Menge (w/w = Gewicht pro Gewicht) des verdünnungsmittelfreien Rohproduktes. Die Polyethermenge liegt bevorzugt zwischen der 0.1fachen und 4fachen Menge (w/w) des Rohproduktes, besonders bevorzugt zwischen der 0.15fachen und 0.4fachen (w/w) Rohproduktmenge.

Die erfindungsgemäß verwendeten Polyether sind bekannt und käuflich.

Die Temperatur des Destillationssumpfes kann beim erfindungsgemäßen Verfahren innerhalb eines größeren Bereichs variiert werden. Im allgemeinen arbeitet man zwischen 60°C und 150°C, bevorzugt bei Sumpf-Temperaturen zwischen 70°C und 120°C, besonders bevorzugt zwischen 90°C und 110°C.

Das erfindungsgemäße Verfahren wird unter vermindertem Druck durchgeführt. Der Destillationsdruck liegt bevorzugt im Bereich zwischen 0.5 mbar und 10 mbar. Besonders bevorzugt arbeitet man zwischen 1 mbar und 4 mbar.

CCMT kann beispielsweise nach den in EP-B 0 446 913 und EP-B 0 763 531 beschriebenen Verfahren hergestellt werden. Das erhaltene Rohprodukt wird zunächst von eventuell noch vorhandenem Verdünnungsmittel befreit. Beim erfindungsgemäßen Verfahren geht man dann im allgemeinen so vor, dass man einen oligomeren Polyether zugibt, anschließend den Druck absenkt und das CCMT dann bei der entsprechenden Temperatur destilliert. Erfindungsgemäß ist es aber auch möglich den oligomeren Polyether bereits vor dem Entfernen vorhandener Verdünnungsmittelreste aus der Synthese zuzugeben.

Bei der kontinuierlichen Durchführung des erfindungsgemäßen Verfahrens wird in einer ersten_Destillationsstufe das Verdünnungsmittel entfernt und der CCMT-haltige Destillationssumpf einer zweiten Destillationsstufe zugeführt, aus der nach Absenken des Druckes CCMT destilliert wird. Dabei kann bei dem erfindungsgemäßen Verfahren der oligomere Polyether entweder bereits in der ersten Stufe oder erst in der zweiten Stufe zugegeben werden.

Bei der diskontinuierlichen Durchführung des erfindungsgemäßen Verfahrens wird das verdünnungsmittelhaltige Rohprodukt mit oligomerem Polyether versetzt. Anschließend wird als erstes das Verdünnungsmittel entfernt, anschließend der Druck abgesenkt und das CCMT destilliert.

CCMT wird beispielsweise als Zwischenprodukt für die Herstellung von Insektiziden verwendet (vgl. EP-A 0 376 279).

### Beispiel

### Synthese

500 g (3,74 mol) 2-Chlorallylisothiocyanat werden in 1250 g Acetonitril gelöst. Bei 10-15°C werden 282 g (3,98 mol) Chlor eingeleitet und bei 20-25°C 2 Stunden nachgerührt. Anschließend wird der Ansatz bei 30-35°C im Vakuum entgast.

Man erhält 1944 g einer 29,3%igen Lösung (GC, interner Standard) von CCMT in Acetonitril (Ausbeute: 91 % d.Th.).

Der Ansatz wird in zwei gleiche Teile geteilt.

### Teil a): Destillation ohne Zusatz

Nachdem das Acetonitril im Vakuum (ca. 350 mbar) abdestilliert wurde, wird der Druck auf 0,6 - 0,7 mbar abgesenkt und CCMT wird bei einer Kopftemperatur von ca. 75°C bis zu einer Sumpftemperatur von 110°C überdestilliert.

Man erhält 263,1 g orange-gelbes Destillat (94,3%ig, GC, interner Standard).

Ausbeute: 87 % d. Th., bezogen auf die in der Destillation eingesetzte Menge.

Der schwarze Sumpf (41,2 g), der beim Abkühlen glasartig fest wird, besitzt einen Gehalt von 5,9 % (GC, interner Standard) CCMT entsprechend einer theoretischen Ausbeute von weiteren 0,9 %.

### Teil b): Destillation mit Zusatz von Polyether

Nachdem das Acetonitril im Vakuum (ca. 350 mbar) abdestilliert wurde, wird zum rohen CCMT 43 g Polyethylenglykol der mittleren Molmasse 400 zugegeben.

Der Druck wird weiter abgesenkt auf 1-2 mbar und CCMT wird bei einer Kopftemperatur von ca. 75°C bis zu einer Sumpftemperatur von 110°C überdestilliert.

Man erhält 268,2 g CCMT als fast farbloses Destillat mit einem Gehalt von 98,5 % (GC, interner Standard).

Ausbeute: 93 % d. Th., bezogen auf die in der Destillation eingesetzte Menge.

Der schwarze Sumpf (79,1 g) bleibt bis Raumtemperatur flüssig. Er besitzt einen Gehalt von 13,7 % (GC, interner Standard) CCMT entsprechend einer theoretischen Ausbeute von weiteren 3,8 %.

## Patentansprüche

1. Verfahren zur Reinigung von 2-Chlor-5-chlormethyl-thiazol, **dadurch gekennzeichnet, dass** man Rohmaterial der Verbindung in Anwesenheit eines Polyethers unter vermindertem Druck destilliert.

2. Verfahren gemäß Anspruch 1, **dadurch gekennzeichnet, dass** der Polyether Polyethylenglykol oder Polypropylenglykol ist.

3. Verfahren gemäß einem der Ansprüche 1 und 2, **dadurch gekennzeichnet, dass** der Polyether eine mittlere Molmasse zwischen 200 und 3000 Dalton hat.

4. Verfahren gemäß einem der Ansprüche 1 und 2, **dadurch gekennzeichnet, dass** der Polyether eine mittlere Molmasse zwischen 300 und 600 Dalton hat.

5. Verfahren gemäß einem der Ansprüche 1 und 2, **dadurch gekennzeichnet, dass** der Polyether eine mittlere Molmasse von 400 Dalton hat.

6. Verfahren gemäß Anspruch 1, **dadurch gekennzeichnet, dass** man bei einem Druck zwischen 0,5 mbar und 10 mbar arbeitet.

7. Verfahren gemäß Anspruch 1, **dadurch gekennzeichnet, dass** man bei einem Druck zwischen 1 mbar und 4 mbar arbeitet.

8. Verfahren gemäß Anspruch 1, **dadurch gekennzeichnet, dass** man bei einer Temperatur zwischen 60 und 150°C arbeitet.

9. Verfahren gemäß Anspruch 1, **dadurch gekennzeichnet, dass** man bei einer Sumpftemperatur zwischen 70°C und 120°C arbeitet.

10. Verfahren gemäß Anspruch 1, **dadurch gekennzeichnet, dass** die zugesetzte Polyethermenge zwischen der 0,01-fachen und 10-fachen Menge (w/w) des verdünnungsmittelfreien Rohproduktes liegt.

11. Verfahren gemäß Anspruch 1, **dadurch gekennzeichnet, dass** die zugesetzte Polyethermenge zwischen der 0,1-fachen und 4-fachen Menge (w/w) des verdünnungsmittelfreien Rohproduktes liegt.

## Claims

1. Process for purifying 2-chloro-5-chloromethyl-thiazole, **characterized in that** crude material of the compound is distilled under reduced pressure in the presence of a polyether.

2. Process according to Claim 1, **characterized in that** the polyether is polyethylene glycol or polypropylene glycol.

3. Process according to Claim 1 or 2, **characterized in that** the polyether has an average molar mass between 200 and 3000 daltons.

4. Process according to Claim 1 or 2, **characterized in that** the polyether has an average molar mass between 300 and 600 daltons.

5. Process according to Claim 1 or 2, **characterized in that** the polyether has an average molar mass of 400 daltons.

6. Process according to Claim 1, **characterized in that** the process is carried out at a pressure between 0.5 mbar and 10 mbar.

7. Process according to Claim 1, **characterized in that** the process is carried out at a pressure between 1 mbar and 4 mbar.

8. Process according to Claim 1, **characterized in that** the process is carried out at a temperature between 60 and 150°C.

9. Process according to Claim 1, **characterized in that** the process is carried out at a bottom temperature between 70 and 120°C.

10. Process according to Claim 1, **characterized in that** the amount of polyether that is added is between 0.01 and 10 times the amount (w/w) of the diluent-free crude product.

11. Process according to Claim 1, **characterized in that** the amount of polyether that is added is between 0.1 and 4 times the amount (w/w) of the diluent-free crude product.

## Revendications

1. Procédé de purification du 2-chloro-5-chloro-méthylthiazole, **caractérisé en ce qu'**on distille la matière brute contenant le composé en présence d'un polyéther sous pression réduite.

2. Procédé suivant la revendication 1, **caractérisé en ce que** le polyéther est un polyéthylèneglycol ou un polypropylèneglycol.

3. Procédé suivant l'une des revendications 1 et 2, **caractérisé en ce que** le polyéther a une masse molaire moyenne comprise entre 200 et 3000 daltons.

4. Procédé suivant l'une des revendications 1 et 2, **caractérisé en ce que** le polyéther a une masse molaire moyenne comprise entre 300 et 600 daltons.

5. Procédé suivant l'une des revendications 1 et 2, **caractérisé en ce que** le polyéther a une masse molaire moyenne de 400 daltons.

6. Procédé suivant la revendication 1, **caractérisé en ce qu'**on opère à une pression comprise entre 0,5 mbar et 10 mbar.

7. Procédé suivant la revendication 1, **caractérisé en ce qu'**on opère à une pression comprise entre 1 mbar et 4 mbar.

8. Procédé suivant la revendication 1, **caractérisé en ce qu'**on opère à une température comprise entre 60 et 150°C.

9. Procédé suivant la revendication 1, **caractérisé en ce qu'**on opère à une température au bas de la colonne comprise entre 70°C et 120°C.

10. Procédé suivant la revendication 1, **caractérisé en ce que** la quantité ajoutée de polyéther est comprise entre 0,01 et 10 fois la quantité (en p/p) du produit brut sans le diluant.

11. Procédé suivant la revendication 1, **caractérisé en ce que** la quantité utilisée de polyéther est comprise entre 0,1 et 4 fois la quantité (en p/p) du produit brut sans le diluant.
